# EUROPÄISCHE PATENTSCHRIFT

(11) **EP 3 171 908 B1**
(45) Veröffentlichungstag und Bekanntmachung des Hinweises auf die Patenterteilung: **11.11.2020**
(21) Anmeldenummer: 15744120.5
(22) Anmeldetag: 09.07.2015
(51) Int. Cl.: A61M 1/10

(54) **ROTATIONSKOLBENPUMPE**
ROTARY PISTON PUMP
POMPE À PISTON ROTATIF

(30) Priorität: 23.07.2014 DE 102014010745
(43) Veröffentlichungstag der Anmeldung: 31.05.2017
(73) Patentinhaber: Rheinisch-Westfälische Technische Hochschule Aachen, 52062 Aachen (DE)
(72) Erfinder: WAPPENSCHMIDT, Johannes Friedrich Wilhelm Gerhard Sönke, 52066 Aachen (DE); GOETZENICH, Andreas Franz Wilhelm, 52066 Aachen (DE); AUTSCHBACH, Rüdiger, 52074 Aachen (DE); STEINSEIFER, Ulrich, 4730 Hauset (BE)
(74) Vertreter: Cohausz Hannig Borkowski Wißgott
(86) Internationale Anmeldenummer: PCT/EP2015/001411
(87) Internationale Veröffentlichungsnummer: WO 2016/012082

(56) Entgegenhaltungen:
- WO-A1-2014/138155
- GB-A- 2 477 276
- US-A- 5 895 421

## Beschreibung

Die Erfindung betrifft eine Rotationskolbenpumpe, insbesondere zur Förderung von Blut, umfassend einen Kolbenläufer, der exzentrisch rotierend angetrieben in einem Kolbenraum angeordnet ist. Eine solche Rotationskolbenpumpe, wie sie den Gegenstand der Erfindung bildet, kann z.B. nach dem Prinzip der Kreiskolbenmaschinen arbeiten, bei der lediglich der Kolbenläufer bewegt ist und der Kolbenraum ortsfest ist (siehe z.B. US5895421).

Bei einer solchen Rotationskolbenpumpe kann beispielsweise der Kolbenläufer mehrkeulig, insbesondere zwei- oder bevorzugt dreikeulig ausgebildet sein, wobei weiterhin der Querschnitt des Kolbenraumes, betrachtet senkrecht zur axialen Erstreckung der Rotationskolbenpumpe an die exzentrische Bewegungsbahn des Kolbenläufers angepasst ist.

Hierzu kann beispielsweise der Kolbenläufer trochoidenförmig ausgebildet sein und der Kolbenraum bildet in seinem Querschnitt bzw. der Kontur der Mantelfläche, die dem Kolbenläufer gegenüberliegt, die korrespondierende Hüllfigur zu dieser Trochoide. Ebenso kann der Querschnitt des Kolbenraumes bzw. die Mantelflächenkontur eine Trochoide beschreiben und der Kolbenläufer bildet mit seinem Querschnitt die korrespondierende Hüllfigur.

Der Begriff "zentrisch" bedeutet innerhalb der vorliegenden Erfindungsbeschreibung eine Lage koaxial bzw. auf der Mittenachse des Kolbenraumes. Unter dem Begriff "exzentrisch" wird dementsprechend eine Lage neben der Mittenachse des Kolbenraumes verstanden.

Rotationskolbenpumpen der vorgenannten Bauart sind im Stand der Technik allgemein bekannt, auch auf dem Gebiet der Blutförderung, somit also beispielsweise als Kunstherz. Beispielsweise offenbart die DE 3317156 eine solche Blutpumpe.

Die Drehbewegung des Kolbenläufers in einer solchen Rotationskolbenpumpe wird in üblicher Ausgestaltung mit Hilfe eines Exzenters übertragen. Dabei erfolgt nach dem bekannten Stand der Technik die Führung des Kolbenläufers auf seiner Bewegungsbahn im Kolbenraum mithilfe eines Zahnradgetriebes.

Die mechanischen Komponenten des Exzenters, insbesondere des Zahnradgetriebes sind gemäß dem bekannten Stand der Technik medienberührt d.h. in Kontakt mit dem geförderten Fluid wie beispielsweise Blut oder es sind alternativ Dichtungen erforderlich, um eine Trennung der mechanischen Komponenten von geförderten Fluid zu erzielen. Bislang stellt jedoch die Medienberührung oder aber die hohen Anforderungen an die Dichtigkeit auf Dauer noch nicht gelöste Probleme bei solchen Rotationskolbenpumpen dar.

Insbesondere im Bereich der Anwendung solcher Rotationskolbenpumpen als Blutpumpe stellt die Kontaktierung der bewegten Exzenterkomponenten des Standes der Technik mit dem Blut oder einem Mangel der Dichtigkeit erhebliche Probleme dar, da es hierbei zu Verklumpungen des Blutes und somit zu einer Gefahr von Thrombenbildung kommen kann. Blutablagerungen, die z.B. trotz eingesetzter Dichtungen entstehen, können auch zu einem Versagen der Pumpe selbst führen.

Es ist daher eine wesentliche Aufgabe der Erfindung eine Rotationskolbenpumpe der eingangs genannten Art bereit zu stellen, bei der die antreibenden und bevorzugt auch angetrieben Komponenten, bevorzugt sämtliche den Antrieb und die Führung der bewegten Teile bewirkenden Elemente, abgesehen vom Kolbenläufer selbst keine Medienberührung, insbesondere also keine Berührung mit Blut aufweisen und somit eine hermetische Dichtigkeit der Pumpenkomponenten gegenüber dem gepumpten Fluid, insbesondere Blut erzielt wird.

Diese Aufgabe wird gemäß der Erfingung, wie beansprucht, dadurch gelöst, dass der Kolbenläufer in seinem Inneren mehrere magnetisch wechselwirkende Elemente aufweist und durch magnetische Wechselwirkungen dieser Elemente mit wenigstens einem bewegten magnetischen Feld angetrieben ist. Diese magnetisch wechselwirkenden Elemente im Inneren des Kolbenläufers können beispielsweise Permanentmagnete und/oder Spulen sein.

Die Erfindung kann hier bevorzugt vorsehen, dass der Kolbenläufer in seinem Querschnitt senkrecht zur Mittenachse eine Hypotrochoidenform aufweist und der Querschnitt des Kolbenraumes bzw. die Kontur der Mantelfläche die korrespondierende Hüllfigur bildet, insbesondere unter Berücksichtigung eines spaltbildenden Versatzes. Die Erfindung ist jedoch auf diese bevorzugte Ausführung nicht beschränkt.

Die Erfindung kann, insbesondere bei der Anwendung als Blutpumpe, weiterhin vorsehen, dass der Kolbenraum nicht nur einen Ein- und Auslass aufweist, sondern zwei Paare von jeweils einander zugeordneten Ein- und Auslässen, z.B. zwischen denen jeweils einer der beiden Körperblutkreisläufe ausgebildet werden kann. Bei der erfindungsgemäßen Rotationskolbenpumpe können Dichtungen, z.B. als Spaltdichtungen in/an dem die Hüllfigur bildenden Element, hier bevorzugt der Mantelfläche des Kolbenraumes ausgebildet sein. Durch zwei feststehende Spaltdichtungen zwischen Gehäuse und Kolbenläufer können so z.B. die beiden Kreisläufe voneinander getrennt werden.

Es kann dabei in einer Ausführung vorgesehen sein, dass die rotierte Bewegung des Kolbenläufers auf einer exzentrischen Bewegungsbahn um die Kolbenraummittenachse herum ausschließlich durch die magnetische Wechselwirkung dieser vorgenannten Elemente mit dem wenigstens einen magnetischen Feld erfolgt. Eine andere Ausführung kann vorsehen, dass die rotierte exzentrische Bewegung zwar durch die magnetische Wechselwirkung initiiert ist, die exzentrische rotierte Bewegung jedoch weiterhin einer Zwangsführung unterliegt, z.B. durch eine mechanische exzentrische Lagerung des Kolbenläufers im Kolbenraum, die bevorzugter Weise ebenso magnetisch realisiert ist.

Eine solche, bevorzugt magnetische exzentrische Lagerung des Kolbenläufers, um die exzentrische Bewegungsbahn des Kolbenläufers zu definieren, kann beispielsweise dadurch realisiert sein, dass der Kolbenläufer an wenigstens einem seiner axialen Enden, bevorzugt an den beiden gegenüberliegenden axialen Enden eine solche Lagerung aufweist.

Dies hat den Vorteil, dass eine solche Lagerung axialseitig ebenso in nicht medienberührten Bereichen der Pumpe stattfinden kann, d.h. außerhalb solcher Bereiche zwischen der inneren Mantelfläche des Kolbenraumes und der äußeren Mantelfläche des Kolbenläufers, die jeweils mit dem geförderten Medium, wie beispielsweise Blut, in Kontakt stehen.

Beispielsweise können hierfür zwischen axialseitigen Stirnflächen des Kolbenraumes und axialseitigen Stirnflächen des Kolbenläufers entweder Dichtungselemente vorgesehen sein oder aber die axialseitig einander gegenüberliegenden Flächenbereiche von Kolbenläufer und Kolbenraum weisen ein derart geringes Bewegungsspiel auf, dass ein Durchtritt des geförderten Mediums, wie beispielsweise Blut, durch diese geringen Spaltbereiche unterbleibt. Eine andere Möglichkeit kann vorsehen, in dem Bereich zwischen den axial gegenüberliegenden Flächenbereichen von Kolbenläufer und Kolbenraum einen Sekundärstrom von dem geförderten Medium zu realisieren, also z.B. von Blut. Ein Sekundärstrom kann alternativ oder ergänzend auch durch die genannte bevorzugt magnetisch wirkende Lagerung des Kolbenläufers hindurch erfolgen.

Durch die Bewegung des wenigstens einen erzeugten Magnetfeldes werden demnach die magnetisch wechselwirkenden Elemente des Kolbenläufers aufgrund der wirkenden Anziehungs- oder Abstoßungskräfte zwischen diesen und dem erzeugten wenigstens einem Magnetfeld mit der Bewegung des Magnetfeldes mitgeführt und so der Kolbenläufer um die Kolbenraummittenachse exzentrisch rotiert.

Insbesondere kann es hierfür vorgesehen sein, dass das bewegte wenigstens eine Magnetfeld eine exzentrische Bewegung um die Mittenachse des Kolbenraumes durchführt und demnach der Kolbenläufer mit seinen daran angeordneten mehreren magnetisch wechselwirkenden Elementen in gleicher Weise diese exzentrische Bewegung vollzieht.

In einem solchen Fall sind bevorzugter Weise die im Inneren des Kolbenläufers angeordneten mehreren magnetisch wechselwirkenden Elemente ortsfest im Kolbenläufer, insbesondere drehfest in diesem angeordnet.

Eine andere Ausführung kann es auch vorsehen, dass das wenigstens eine bewegte Magnetfeld unabhängig vom Ort seiner Erzeugung, wie beispielsweise außerhalb des Kolbenraumes, eine zentrische Bewegung um die Kolbenraummittenachse vollzieht und diese zentrische Bewegung auf die mehreren magnetisch wechselwirkenden Elemente des Kolbenläufers übertragen wird, die hingegen bei einer solchen Ausführung zentrisch rotierbar im Kolbenläufer gelagert sind. So wird die zentrische Bewegung der magnetisch wechselwirkenden Elemente um die Kolbenraummittenachse herum durch eine exzentrische Lagerung dieser Elemente relativ zum Kolbenläufer in eine exzentrische Bewegung des Kolbenläufers innerhalb des Kolbenraumes umgesetzt.

Eine mögliche, insbesondere ortsfeste Anordnung der magnetisch wechselwirkenden Elemente im Kolbenläufer kann beispielsweise dadurch erfolgen, dass diese Elemente im Bereich der Keulenenden des Kolbenläufers angeordnet sind. Hierbei können weiterhin in Umfangsrichtung außen um die innere Mantelfläche des Kolbenraumes herum mehrere zur Erzeugung des bewegten Magnetfeldes abwechselnd bestrombare Spulen angeordnet sein, wobei die eingangs genannten magnetisch wechselwirkenden Elemente des Kolbenläufers und die Spulen einander radial (bezogen auf die Mittenachse des Kolbenraumes) gegenüber liegen, die magnetische Anziehung oder Abstoßung zwischen diesen also im Wesentlichen radial erfolgt.

Durch eine abwechselnde Bestromung der in Umfangsrichtung angeordneten Spulen wird demnach bewirkt, dass das Magnetfeld ebenso entlang des Umfanges des Kolbenraumes wandert und durch die magnetische, insbesondere anziehende Wechselwirkung die magnetisch wechselwirkenden Elemente, wie beispielsweise Permanentmagnete, im Kolbenläufer in der Bewegungsrichtung des Magnetfeldes mitbewegt werden.

Die magnetisch wechselwirkenden Elemente im Kolbenläufer, insbesondere darin ortsfest angeordnete Elemente, sind im Bereich wenigstens eines axialen Endes in oder unter einer axialen Stirnfläche des Kolbenläufers angeordnet. Die Magnetfeldlinien dieser Elemente durchdringen somit bevorzugt im Wesentlichen die axiale Stimfläche des Kolbenläufers und/oder des Kolbenraumes.

Sodann ist vorgesehen, dass die zur Erzeugung des bewegten Magnetfeldes abwechseln bestrombare Spulen axial beabstandet zu den Elementen des Kolbenläufers hinter einer axialen Stirnwand des Kolbenraumes angeordnet sind, wobei diese Spulen korrespondierend zur Kontur, der Mantelfläche des Kolbenraumes (ggfs. um einen Offset versetzt) oder korrespondierend zur Bewegungsbahn der magnetisch wechselwirkenden Elemente im Kolbenläufer angeordnet sind, insbesondere in letzterem Fall die Bewegungsbahn überdecken. Wird demnach eine abwechselnde Bestromung dieser Spulen vorgenommen, so wandert das wenigstens eine bewegte Magnetfeld bevorzugt in gleicher Weise wie die magnetisch wechselwirkenden Elemente, wobei hier jedoch magnetische Anziehungskräfte oder Abstoßungskräfte zwischen den Spulen und den Elementen des Kolbenläufers axial gerichtet sind.

Besonders bei einer axial beidseitigen Konstruktion dieser vorgenannten Ausführung wird eine verbesserte axiale Lagestabilität des Kolbenläufers im Kolbenraum erzielt. Ggfs. kann es auch vorgesehen sein, bei dieser Ausführung, aber auch allen anderen Ausführungen mit bestromten Spulen zur Realisierung des Antriebes eine aktive Regelung der Spulenströme vorzunehmen, um die Lage, z.B. axiale und/oder radiale Lage oder Winkellage des Kolbenläufers im Kolbenraum zu stabilisieren.

Eine andere Ausführung in Zusammenhang mit der Positionierung der magnetisch wechselwirkenden Elemente im Kolbenläufer im Bereich wenigstens eines axialen Endes, insbesondere in oder unter einer axialen Stirnfläche desselben kann vorsehen, dass hinter einer axialen Stirnwand des Kolbenraumes, insbesondere welche demnach innenseitig zumindest zum Teil medienberührt ist, mehrere Permanentmagnete axial beabstandet zu den magnetischen Elementen des Kolbenläufers auf einem rotierbaren Rotor angeordnet sind, der durch einen Motor angetrieben oder zumindest antreibbar ist.

So kann durch den motorischen Antrieb eines solchen Rotors demnach ein bewegtes magnetisches Feld erzeugt werden, welches durch die axiale Stirnwandung des Kolbenraumes in das Innere des Kolbenraumes und somit auf die magnetischen Elemente des Kolbenläufers wirkt, somit also ebenso der Kolbenläufer durch die ausschließliche magnetische Wechselwirkung angetrieben ist.

Auch hier ist es ersichtlich, dass die mechanischen Komponenten des Antriebes, hier der rotierte Rotor mit Permanentmagneten keinerlei Medienberührung aufweist, da dieser Rotor außerhalb des Kolbenraumes, z.B. hinter einer axialen Stirnwand des Kolbenraumes angeordnet ist.

Bei einer ortfesten Anordnung der wechselwirkenden magnetischen Elemente im Kolbenläufer kann es hier vorgesehen sein, dass der rotierbare Rotor eine exzentrische Rotation bzgl. der Mittenachse des Kolbenraumes durchführt, so dass sich die exzentrische Bewegung dieses Rotors auf diejenige des Kolbenläufers überträgt. Unter der exzentrischen Rotation des Rotors oder auch bei anderen im Rahmen dieser Erfindung beschrieben Elementen wird verstanden, dass sich das betrachtete Element um sich selbst dreht, insbesondere um den eigenen Schwerpunkt und dabei insbesondere dieser Schwerpunkt um die Mittenachse des Kolbenraumes umläuft, also zwei Drehbewegungen überlagert sind.

Ein solcher Rotor kann alternativ auch zentrisch um die Mittenachse des Kolbenraumes rotierend angeordnet sein. Bei einer solchen Ausführung wird es jedoch dann vorgesehen sein, dass die magnetisch wechselwirkenden Elemente des Kolbenläufers in diesem rotierbar an einem Exzenter gelagert sind, demnach also der Exzenter, der diese Elemente trägt eine zentrische Rotationsbewegung um die Kolbenraummittenachse durchführt und aufgrund seiner exzentrischen Form und exzentrischen Lagerung im Kolbenläufer diesen sodann auf eine exzentrische Bewegungsbahn zwingt.

Wie bereits eingangs genannt kann es jede der vorangehend diskutierten Ausführungen sowie auch die nachfolgend noch zu diskutierenden Ausführungen ergänzend vorsehen, dass der Kolbenläufer an wenigstens einem seiner axialen Enden, bevorzugt an beiden axialen Enden exzentrisch rotierbar gelagert ist, insbesondere magnetisch gelagert ist.

Eine solche Lagerung kann beispielsweise ausgebildet sein durch einen ersten magnetischen oder zumindest magnetisierbaren zentrisch an einer axialen Kolbenraumwand befestigten Ring, dessen Ringmitte demnach also auf der zentrischen Kolbenraummittenachse liegt und durch einen am axialen Ende im Kolbenläufer exzentrisch angeordneten zweiten magnetischen oder zumindest magnetisierbaren Ring, dessen Ringmitte also neben der Kolbenraummittenachse angeordnet ist, wobei dieser zweite Ring bei der Rotation des Kolbenläufers mit seiner inneren Ringfläche unter magnetisch anziehender Wechselwirkung unmittelbar oder mittelbar über ein zwischen den Ringflächen angeordnetes Material, insbesondere ein magnetisierbares Material auf der äußeren Ringfläche des ersten Ringes abrollt. Ein solches zwischengefügtes Material kann z.B. Polschuhe, insbesondere blutverträgliche Polschuhe ausbilden. Dabei kann es vorgesehen sein durch eine mechanische Bearbeitung dieses Materials die Abrolleigenschaften der Ringe zu verbessern. Das zwischengefügte Material kann bei allen möglichen Ausführungen auch als eine Beschichtung wenigstens einer der sich gegenüberliegenden Ringoberflächen ausgebildet sein.

Eine magnetisch anziehende Wechselwirkung zwischen innerer und äußerer Ringfläche dieser beiden vorgenannten Ringe kann beispielsweise dadurch realisiert sein, dass einer der Ringe, bevorzugt beide Ringe in ihrer jeweiligen Umfangsrichtung abwechselnd magnetisiert sind, so dass in radialer Richtung betrachtet, beim Vorgang des Abrollens sich jeweils anziehende Polungen der Ringmagnetisierungen gegenüberliegen. Hierdurch wird quasi eine Art magnetisches Zahnrad realisiert, bei dem in Umfangsrichtung jeweils abwechselnd Nord- und Südpol in magnetischen Eingriff zueinander gelangen. Eine solche Konstruktion kann wirkungsvoll ein Rutschen der Ringe aufeinander verhindern.

Eine andere Ausführung kann auch vorsehen, dass wenigstens einer der Ringe, bevorzugt beide, in radialer Richtung ihre jeweilige im Umfangsrichtung gleichbleibende magnetische Polung aufweisen, also radial innenliegend zum Beispiel den Nordpol und radial außenliegend den Südpol, so dass bei einer solchen Anordnung in Umfangsrichtung betrachtet immer die beiden gleichen gegensätzlichen Polungen aufeinander abrollen. Ebenso können axial magnetisierte Ringe verwendet werden.

Zu den Ausführungen mit den vorgenannten beiden Ringen ist anzumerken, dass zwar bevorzugt beide Ringe magnetisiert sein können, es für die erfindungsgemäße Wirkung jedoch ausreichend ist, wenn einer der Ringe magnetisiert und der andere lediglich magnetisierbar ist.

Durch das Abrollen von innerer und äußerer Ringfläche der beiden vorgenannten beiden Ringe wird demnach der Kolbenläufer in seiner exzentrischen Bewegung zwangsgeführt, so dass die Bewegung des Kolbenläufers in Folge des wenigstens einen bewegten erzeugten Magnetfeldes keinerlei Risiken einer axialen Verkippung oder radial ungewünschten Verschiebung unterliegt.

Wie bereits eingangs erwähnt, kann grundsätzlich durch die bevorzugt axial endseitige Anordnung der Lagerung insbesondere in einer Ausnehmung in einer axialen Stirnfläche des Kolbenläufers erzielt werden, dass auch diese Lagerung keine Medienberührung aufweist, sofern das geförderte Medium, beispielsweise Blut, nicht den axialseitigen Spalt zwischen Kolbenläufer und Kolbenraumwandung durchdringen kann, was beispielsweise durch entsprechend geringe Spaltmaße oder zusätzliche Dichtungen erzielt werden kann.

Diese Ringen können alternativ zur Ausbildung einer bereits zuvor beschriebenen Sekundärströmung auch von dem geförderten Medium, insbesondere Blut umströmt sein. In einem solchen Fall können Dichtungsmaßnahmen entfallen, was die Konstruktion vereinfacht und ausfallsicherer gestaltet. Hierbei ist die genannte Lagerungsausführung besonders vorteilhaft, da zwischen den Ringen nur ein Linienkontakt beim Abrollen entsteht und somit keine Blutschädigungen zu erwarten sind.

Eine weitere Ausführung kann vorsehen, dass die magnetisch wechselwirkenden Elemente in einem im Kolbenläufer zentrisch relativ zum Kolbenraummittenachse rotierbar gelagerten Exzenter angeordnet sind. Diese Ausführung stellt darauf ab, dass das wenigstens eine bewegte magnetische Feld zum Antrieb des Kolbenläufers eine zentrische Bewegung um die Kolbenraummittenachse durchführt, demnach die magnetisch wechselwirkenden Elemente des Kolbenläufers auf dieser zentrischen Bewegung mitgeführt werden und die exzentrische Bewegung des Kolbenläufers dadurch erzeugt wird, dass der Exzenter im Kolbenläufer um die Kolbenraummittenachse zentrisch rotierbar gelagert ist. Die Exzentrizität dieses Exzenters bei seiner zentrisch rotierenden Bewegung überträgt sich demnach auf die Bewegung des Kolbenläufers.

Sowohl bei dieser Ausführungsform als auch bei allen anderen möglichen Ausführungsformen kann es vorgesehen sein, dass die mehreren wechselwirkenden Elemente des Kolbenläufers, sofern diese als Permanentmagnete ausgebildet sind, durch einen permanentmagnetischen Ring gebildet sind, der in Umfangsrichtung mehrfach abwechselnd magnetisiert ist. Für die Erfindung ist es demnach gleichwirkend, ob mehrere einzelne Permanentmagnete, insbesondere in einer kreisförmigen Anordnung, im Kolbenläufer, in oder unter einer axialseitigen Stirnseite des Kolbenläufers angeordnet sind oder ob stattdessen ein ringförmiger Permanentmagnet mit solcher in Umfangsrichtung abwechselnden Magnetisierung eingesetzt wird.

Ebenso bei den zuvor genannten Rotoren für die Erzeugung bewegter Magnetfelder kann es vorgesehen sein, statt einzelner beispielsweise kreisförmig angeordneter Permanentmagnete ebenso hier auch einen permanentmagnetischen Ring zu verwenden, der diese besagte in Umfangsrichtung mehrfach abwechselnd gepolte Magnetisierung aufweist.

In Verbindung mit einem solchen zentrisch rotierbar gelagerten Exzenter innerhalb des Kolbenläufers kann es vorgesehen sein, dass das wenigstens eine bewegte Magnetfeld auch innerhalb des Kolbenläufers erzeugt sein kann und hierbei beispielsweise durch wenigstens eine den Exzenter umgebende Wandung des Kolbenläufers hindurchwirkt. Eine Erzeugung des wenigstens einen Magnetfeldes innerhalb des Kolbenläufers kann weiterhin derart erfolgen, dass die das Magnetfeld erzeugenden Komponenten außerhalb des Kolbenraumes liegen, der das geförderte Medium umschließt, was beispielsweise dadurch erfolgen kann, dass wenigstens ein Rotor mit darauf bewegten Permanentmagneten in einzelner oder ringförmiger Ausgestaltung von einer axialseitigen Stirnwandung des Kolbenraumes in das Innere des Kolbenläufers vorspringend angeordnet ist. Beispielsweise kann es dafür vorgesehen sein, dass der Exzenter im Kolbenläufer mittig bezogen auf die axiale Länge des Kolbenläufers angeordnet ist und zumindest zu einer Seite des Exzenters, bevorzugt beidseits des Exzenters, weiter bevorzugt hinter einer axialseitigen Wandung des Kolbenläufers, dieser Kolbenläufer eine innere Ausnehmung aufweist, in welcher ein motorisch zentrisch rotierbarer Rotor angeordnet ist, der mehrere zu den magnetisch wechselwirkenden Elementen des Kolbenläufers axial beabstandete Permanentmagnete aufweist.

Auch diese Ausführung kann ergänzend vorsehen, dass axial endseitig des Kolbenläufers, bevorzugt an beiden axialen Endseiten, eine magnetische Lagerung gemäß vorgenannter Ausführung vorhanden ist. Beispielsweise kann diese Ausführung dann weiterhin vorsehen, dass die Rotationswelle des wenigstens einen rotierbaren Rotors sich durch den ersten magnetischen oder zumindest magnetisierbaren zentrisch an einer axialen Kolbenraumwand befestigten Ring einer magnetischen Lagerung des Kolbenläufers hindurch erstreckt.

Alternativ kann es vorgesehen sein, dass am Kolbenläufer axial einseitig, bevorzugt zu beiden axialen Seiten axial vorspringende Antriebselemente angeordnet sind, welche die magnetisch wechselwirkenden Elemente, insbesondere Permanentmagnete tragen und die in Ausnehmungen im Kolbenraum einliegen. Hinter axialseitigen Wandbereichen dieser Ausnehmungen im Kolbenraum können dann jeweils angetriebene Rotoren angeordnet sein. Auch hier kann es vorgesehen sein, dass die magnetisch wechselwirkenden Elemente am Kolbenläufer ortsfest sind und die Rotoren exzentrisch rotiert werden oder umgekehrt die Rotoren zentrisch rotieren und die magnetisch wechselwirkenden Elemente im Kolbenläufer exzentrisch rotierbar auf einem Exzenter angeordnet sind, wie zuvor beschrieben bei der bevorzugt mittigen Anordnung eines Exzenters

Die möglichen Ausführungsvarianten der Rotationskolbenpumpe werden nachfolgend anhand der Figuren diskutiert.

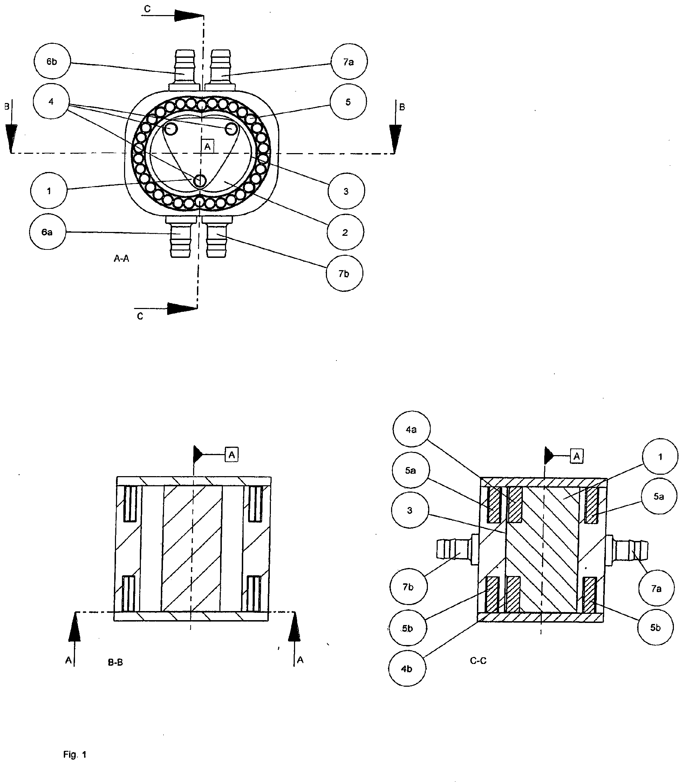

Die Figur 1 zeigt in mehreren Ansichten eine erste Ausführungsform einer Rotationskolbenpumpe. Die Kolbenpumpe weist einen dreikeuligen Kolbenläufer 1 auf, der exzentrisch um die Mittenachse A des Kolbenraumes 2 rotierend bewegt wird. Der Kolbenläufer hat die Form einer Hypotrochoide. Der Kolbenraum 2 weist hier im Querschnitt senkrecht zu seiner Mittenachse A die Form der zugehörigen Hüllfigur auf. Die Kontur der inneren Mantelfläche 3 des Kolbenraumes 2 entspricht also ebenso dieser Hüllfigur, insbesondere unter Berücksichtigung eines Versatzes zur mathematisch berechneten Hüllfigur, um einen Spalt zwischen Kolbenläufer und Kolbenraum zu erzeugen.

Der als dreikeulig bezeichnete Kolbenläufer 1 hat hier im Wesentlichen eine dreieckige Form mit abgerundeten Ecken und leicht ausgebauchten Seiten. Die Querschnittsform senkrecht zur Mittenachse A des Kolbenraumes entspricht bevorzugt der genannten Hypotrochoide.

Die Ausführungsform der Figur 1 weist keinerlei zusätzliche Lagerung des Kolbenläufers 1 auf, das heißt, dieser ist grundsätzlich frei beweglich innerhalb des Kolbenraumes 2 hinsichtlich seiner Rotationsbewegung.

Die exzentrische Rotationsbewegung bzw. Umlaufbewegung des Kolbenläufers kann vorliegend nun dadurch bewirkt werden, dass eine magnetische Kopplung zwischen Kolbenläufer 1 und wenigstens einem außerhalb des Kolbenraumes 2 erzeugten Magnetfeld erfolgt. Hierfür ist es gemäß der Erfindung vorgesehen, dass innerhalb des Kolbenläufers 1 mehrere elektrisch wechselwirkende Elemente 4 angeordnet sind, nämlich vorliegend Permanentmagnete 4 in den Endbereichen der jeweiligen drei Keulen des dreikeuligen Kolbenläufers. Im Wesentlichen liegt also eine Positionierung dieser Elemente nahe der gerundeten Ecken der dreieckförmigen Form vor.

Radial bezogen auf die Mittenachse A des Kolbenraumes 2 sind in Umfangsrichtung um den Kolbenraum 2 herum eine Vielzahl von abwechselnd bestrombaren Spulen 5 angeordnet, so dass durch die abwechselnde Bestromung einzelner Spulen 5 ein in Umfangsrichtung um den Kolbenraum herumwanderndes Magnetfeld oder mehrere wandernde Magnetfelder erzeugt werden können.

Bevorzugt kann es hier vorgesehen sein, dass durch entsprechende Ansteuerung der einzelnen Spulen 5 jeweils wenigstens eine der Spulen 5 mit jedem der magnetisch wechselwirkenden Elemente 4 des Kolbenläufers, hier also drei Spulen 5 oder Spulengruppen mit jeweils einem der drei Elementen 4 in magnetische Wechselwirkung tritt und insbesondere durch anziehende, alternativ durch abstoßende magnetische Wechselwirkung, eine exzentrische Rotation des Kolbenläufers 1 erzeugt wird.

Die linksseitigen Schnittdarstellungen in Richtung der Mittenachse A des Kolbenraumes 2 zeigen weiterhin, dass innerhalb des Kolbenläufers 1 zwei Gruppen von magnetisch wechselwirkenden Elementen 4, nämlich Elemente 4a der ersten Gruppe und Elemente 4b der zweiten Gruppe vorgesehen sind, wobei die Elemente dieser beiden verschiedenen Gruppen in Richtung der Mittenachse A des Kolbenraumes 2 axial beabstandet sind, bevorzugt eine Positionierung dieser Elemente an den jeweiligen axialen Endbereichen des Kolbenläufers 1 vorgesehen sind.

In jeweils radialer Gegenüberlage sind entsprechend bestrombare Spulen 5a beziehungsweise 5b ebenso an den axialseitigen Enden des Kolbenraumes in Umfangsrichtung um den Kolbenraum herum, das heißt der Hüllfigur folgend angeordnet. Es ergeben sich demnach auch hier zwei Gruppen von Spulen, wobei jede Gruppe von wechselwirkenden Elementen 4 des Kolbenläufers genau einer Gruppe von Spulen 5 zugeordnet sind, hier also eine Zuordnung zwischen den Elementen 4a und den Spulen 5a sowie den Elementen 4b und den Spulen 5b gegeben ist.

Durch die axial endseitige Anordnung der beiden Gruppen kann erzielt werden, dass der Kolbenläufer bei seiner exzentrischen Rotation keine oder zumindest keine wesentliche axiale Verkippung erfährt.

Die Figur 1 zeigt weiterhin, dass die hier beschriebene Rotationskolbenpumpe zwei separate Funktionen durchführen kann, nämlich einmal eine Pumpfunktion zwischen dem Einlass 6a und Auslass 6b sowie dem Einlass 7a und dem Auslass 7b. Eine solche Aufteilung ist insbesondere in der Anwendung als Herzpumpe vorteilhaft, da hierdurch separate Lungen- und Körperkreisläufe realisiert werden können. Diese Aufteilung ist jedoch nicht zwingend, es kann auch eine Rotationskolbenpumpe zur Förderung beliebiger Medien, beispielsweise auch Blut, realisiert werden mit lediglich einem Pumpeneinlass und einem Pumpenauslass. Die entsprechenden Realisierungen hängen im Wesentlichen von den konkreten Querschnittsgestaltungen des Kolbenläufers und des Kolbenraumes ab. Bei einer beispielsweisen lediglich zweikeuligen Ausgestaltung des Kolbenläufers wird eine Rotationskolbenpumpe gemäß der Erfindung demnach nur einen Einlass und einen Auslass aufweisen. Z.B. kann mit einer solchen Pumpe nur ein Körperkreislauf unterstützt werden oder es werden alternativ zwei solcher Pumpen eingesetzt.

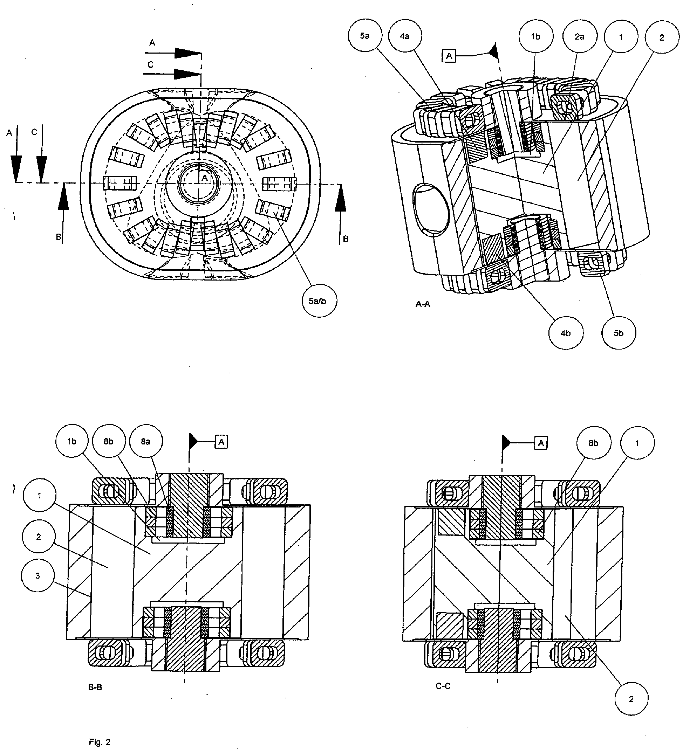

Die Figur 2 zeigt ein alternatives Ausführungskonzept der erfindungsgemäßen Rotationskolbenpumpe, bei welcher magnetisch wechselwirkende Elemente 4a und 4b jeweils wieder in der vorerwähnten Gruppenanordnung an den axialseitigen Stirnflächen des Kolbenläufers 1 angeordnet sind, wobei hier jede Gruppe bevorzugt so wie auch bei der vorherigen Ausführung eine Anzahl von magnetisch wechselwirkenden Elementen aufweist, die der Anzahl der Keulen des Kolbenläufers entspricht. Die magnetisch wechselwirkenden Elemente 4a und 4b können hier beispielsweise in Ausnehmungen der axialen Stirnflächen des Kolbenläufers angeordnet sein.

Eine mögliche, jedoch hier nicht gezeigte Variante kann zusätzliche axiale Permanentmagnete in den Flanken des Kolbenläufers aufweisen, insbesondere also drei an den Enden der Keulen und drei Magnete jeweils dazwischen, bevorzugt an der engsten Stelle zwischen Kolbenläuferaußenwand und Ringmagnet des Kolbenläufers.

Außerhalb des Kolbenraumes, der in axialer Richtung durch axiale Stirnwandungen 2a nach außen hin abgeschlossen ist, sind mehrere Spulen 5, hier wiederum in Gruppenanordnungen, das heißt Spulen 5a bei einer ersten Gruppe und Spulen 5b einer zweiten Gruppe angeordnet, wobei die beiden Gruppen an den beiden axial einander gegenüberliegenden Stirnwandungen des Kolbenraumes angeordnet sind. Es ergibt sich dadurch eine axiale Beabstandung zwischen den Spulen und den magnetisch wechselwirkenden Elementen des Kolbenläufers im Vergleich zu der radialen Beabstandung der Ausführungsform gemäß der Figur 1.

Hier sind die einzelnen Spulen 5a beziehungsweise 5b derart angeordnet, dass diese Anordnung im Wesentlichen der Hüllfigur des trochoidenförmigen Kolbenläufers folgt bzw. die Bewegungsbahn der magnetisch wechselwirkenden Elemente 4a/4b überdeckt ist. Dies bedeutet, dass sich eine optimale axiale Gegenüberlage zwischen jedem magnetisch wechselwirkenden Element 4a beziehungsweise 4b des Kolbenläufers und den zugeordneten Spulen an jedem Ort der exzentrischen Rotationsbewegung des Kolbenläufers ergibt.

Die Ausführungsform der Figur 2 zeigt weiterhin, dass an beiden axialen Enden der erfindungsgemäßen Rotationskolbenpumpe der Kolbenläufer 1 zusätzlich eine Lagerung aufweist, die die exzentrische Bewegung des Kolbenläufers 1 zwangsführt. Diese Lagerung ist hier vorliegend dadurch realisiert, dass zentrisch zur Mittenachse A des Kolbenraumes ein erster permanentmagnetischer Ring 8a vorgesehen ist und exzentrisch zur Achse A ein zweiter Ring 8b, insbesondere so dass bei einer Rotation des Kolbenläufers 1 die innere Ringfläche des äußeren Ringes 8b direkt oder auch indirekt auf der äußeren Fläche des inneren Ringes 8a unter magnetischer anziehender Wirkung abrollt.

Hierbei ist der innere zentrisch mit seiner Ringmitte auf der Achse A positionierte Ring 8a an einem an der axialen Kolbenraumwandung 2a zentrisch positionierten Zapfen befestigt und ragt in eine innere Ausnehmung 1b an der jeweiligen axialen Stirnseite des Kolbenläufers 1 in diesen hinein. Der äußere exzentrisch positionierte Ring 8b, dessen Mitte also radial neben der Achse A liegt, ist in der Ausnehmung 1b des Kolbenläufers 1 befestigt.

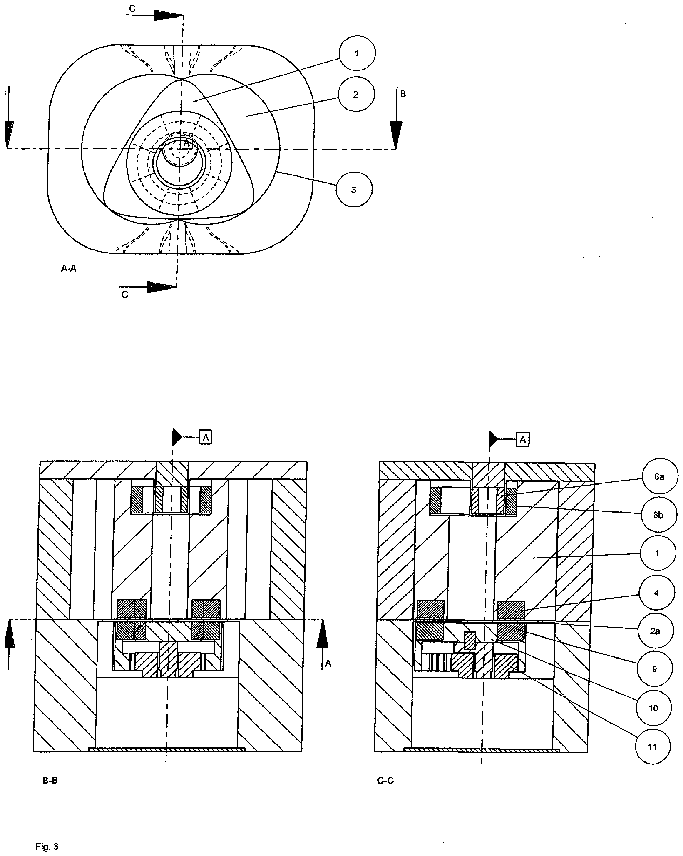

Figur 3 zeigt eine weitere Ausführungsform, bei der, bezogen auf die Figur 3 oben, eine magnetische Lagerung mit innerem ersten und äußerem zweiten magnetischen oder zumindest magnetisierbaren Ringen 8a und 8b und an dem axial gegenüberliegenden, hier also dem unteren Ende, der magnetische Antrieb realisiert ist, wofür wiederum der Kolbenläufer 1 axial stirnseitig positionierte magnetisch wechselwirkende Elemente 4, beispielsweise Permanentmagnete aufweist.

Axial beabstandet und außerhalb des Kolbenraumes 2 ist ein Rotor positioniert, der Permanentmagneten 9 trägt, deren relative Anordnung zueinander derjenigen der magnetisch wechselwirkenden Elemente 4 des Kolbenläufers 1 entspricht, so dass jedem magnetisch wechselwirkenden Element 4 des Kolbenläufers ein Permanentmagnet des Rotors zugeordnet ist.

Der Rotor 10 kann hier vorliegend exzentrisch zur Mittenachse A des Kolbenraumes rotiert werden, so dass die einzelnen Elemente 9, insbesondere die Permanentmagnete demnach eine exzentrisch rotierte Bewegungsbahn aufweisen und den Kolbenläufer mit seinen Elementen 4 mit derselben Bahn mitbewegen. Der Kolbenläufers 1 vollzieht demnach auch hier eine exzentrische Rotation, die ergänzend durch die magnetische Lagerung mit den Ringen 8a und 8b zwangsgeführt ist, welche in der Lagerung dieselbe Exzentrizität aufweisen, wie die Elemente 9 des Rotors 10.

Hier kann es vorgesehen sein, dass der Rotor 10 mittels Zahnrädern 11 auf einer exzentrischen Bewegungsbahn geführt wird. Durch die Zahnräder kann z.B. ein Hohlradgetriebe oder Planetengetriebe ausgebildet sein. Der Antrieb des Rotors kann mit einem üblichen Elektromotor erfolgen, wobei aus der Figur ersichtlich ist, dass sämtliche Komponenten des Rotors außerhalb des Kolbenraumes 2 angeordnet sind und der Antrieb des Kolbenläufers 1 lediglich durch die magnetische Wechselwirkung durch die axial stirnseitige Kolbenraumwandung 2a erfolgt.

Bei der Ausführungsform gemäß der Figur 3 ist die Positionierung der magnetisch wechselwirkenden Elemente 4 des Kolbenläufers 1 ortsfest relativ zum Kolbenläufer.

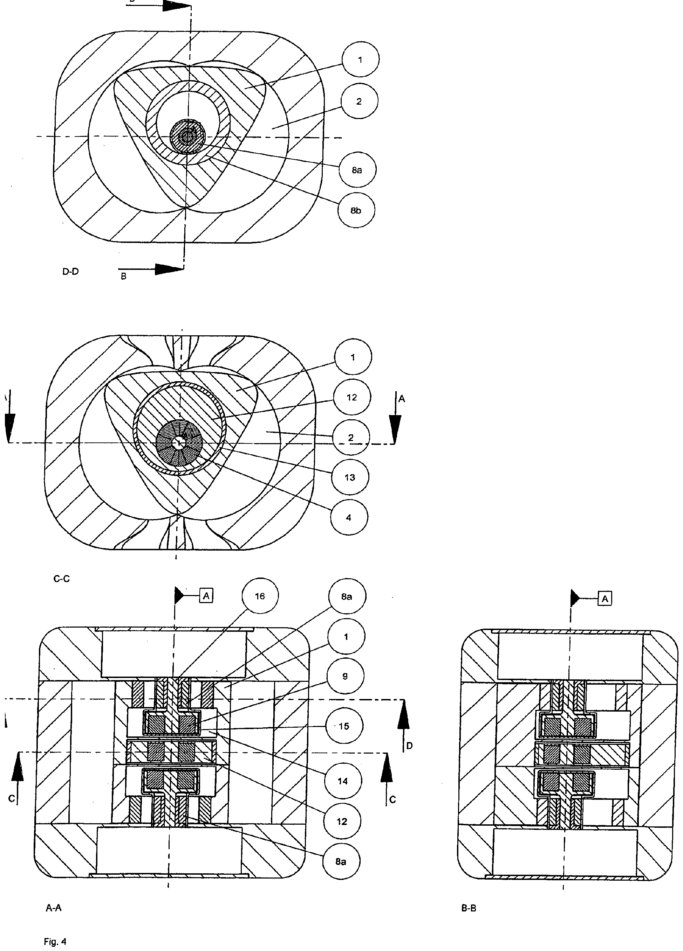

Die Figur 4 beschreibt demgegenüber eine Anordnung, bei welcher im Kolbenläufer 1 die magnetisch wechselwirkenden Elemente 4 als kreisförmig angeordnete Permanentmagnete oder alternativ als permanentmagnetischer Ring zentrisch um die Mittenachse A angeordnet ist, wobei diese Elemente von einem kreisförmigen Exzenter 12 aufgenommen sind, der im Kolbenläufer 1 über eine den Exzenter 12 umgebende Lagerung 13 rotierbar gelagert ist. Die Rotation des Kolbenläufers 1 auf einer exzentrischen Bewegungsbahn erfolgt hier demnach dadurch, dass durch magnetische Wechselwirkung zwischen einem zentrisch, also um die Kolbenraummittenachse A rotierten Rotor mit Permanentmagneten, die Permanentmagneten 4 des Exzenters 12 zentrisch rotiert werden, hierdurch der Exzenter 12 um die Mittenachse A des Kolbenraumes 2 rotiert und aufgrund seiner eigenen Exzentrizität den Kolbenläufer 1 auf eine exzentrische Bewegungsbahn im Kolbenraum zwingt.

Die hier vorliegende Ausführungsform ist weiterhin derart ausgestaltet, dass der Exzenter 12 bezogen auf die Mittenachse A und die axiale Länge des Kolbenläufers 1 mittig im Kolbenläufer 1 positioniert ist, wobei zu beiden Seiten der Mitte innerhalb des Kolbenläufers innere Ausnehmungen 14 vorgesehen sind, in denen Rotoren 15 angeordnet sind, die um die Mittenachse A rotieren und um die Mittenachse A angeordnete magnetisch wechselwirkende Elemente 9, insbesondere Permanentmagnete aufweisen. Hier ist es vorgesehen, dass die Welle 16 der Rotoren 15 durch einen inneren Ring 8a einer magnetischen exzentrischen Lagerung, wie sie bereits zu den vorgenannten Figuren beschrieben wurde, hindurchgeführt ist.

Die Darstellung des axial endseitigen Schnittes in der Figur 4 zeigt hier, dass die innere Ringfläche des äußeren magnetischen Ringes 8b auf der äußeren Ringfläche des inneren zentrisch gelagerten magnetischen Ringes 8a abrollt, so dass durch diese Abrollbewegung die exzentrische Bewegung des Kolbenläufers 1 weiterhin zwangsgeführt ist. Zwischen den Ringen kann ein ringförmiges Zwischenelement angeordnet sein oder wenigstens ein Ring eine Beschichtung tragen, was allgemein für alle möglichen Ausführungen mit einer solche magnetischen Ringlagerung und nicht nur für diese hier dargestellte Ausführung gelten kann.

Zu der Ausführung der Magnetlagerung bei allen vorbeschriebenen Figuren ist weiterhin anzumerken, dass lediglich die Notwendigkeit besteht, dass einer der beiden genannten Ringe magnetisch ist, wohingegen der andere Ring lediglich magnetisierbar ausgeführt sein muss. Alternativ können selbstverständlich beide magnetisch ausgebildet sein. Die Magnetisierungen der Ringe können dabei sowohl radial, axial oder in beliebiger Art derart ausgeführt sein, dass eine anziehende Wechselwirkung zwischen diesen Ringen bei dem Abrollen aufeinander erzielt wird.

## Patentansprüche

1. Rotationskolbenpumpe, insbesondere zur Förderung von Blut, umfassend einen insbesondere im Querschnitt zwei- oder dreikeuligen Kolbenläufer (1), der exzentrisch rotierend angetrieben in einem Kolbenraum (2) angeordnet ist, wobei der Kolbenläufer (1) in seinem Inneren mehrere magnetisch wechselwirkende Elemente (4, 4a, 4b), insbesondere Permanentmagnete und/oder Spulen aufweist und durch magnetische Wechselwirkung dieser Elemente (4, 4a, 4b) mit wenigstens einem bewegten magnetischen Feld angetrieben ist, **dadurch gekennzeichnet, dass** die magnetisch wechselwirkenden Elemente (4) im Kolbenläufer (1) im Bereich wenigstens eines axialen Endes in oder unter einer axialen Stirnfläche angeordnet sind und mehrere zur Erzeugung des bewegten Magnetfeldes abwechselnd bestrombare Spulen (5, 5a, 5b) axial beabstandet zu den magnetisch wechselwirkenden Elementen (4, 4a, 4b) des Kolbenläufers (1) hinter einer axialen Stirnwand des Kolbenraumes (2) angeordnet sind, wobei die Spulen (5, 5a, 5b) korrespondierend zur Querschnittskontur der Mantelfläche (3) oder korrespondierend zur Bewegungsbahn der magnetisch wechselwirkenden Elemente (4, 4a, 4b) des Kolbenläufers (1) angeordnet sind.

2. Rotationskolbenpumpe nach einem der vorherigen Ansprüche, **dadurch gekennzeichnet, dass** der Kolbenläufer (1) an wenigstens einem seiner axialen Enden magnetisch exzentrisch rotierbar gelagert ist.

3. Rotationskolbenpumpe nach Anspruch 2, **dadurch gekennzeichnet, dass** die Lagerung ausgebildet ist durch einen ersten magnetischen oder zumindest magnetisierbaren zentrisch an einer axialen Kolbenraumwand befestigten Ring (8a) und einen am axialen Ende im Kolbenläufer (1) exzentrisch angeordneten zweiten magnetischen oder zumindest magnetisierbaren Ring (8b), der bei Rotation des Kolbenläufers (1) mit seiner inneren Ringfläche unter magnetisch anziehender Wechselwirkung unmittelbar oder mittelbar über ein insbesondere magnetisierbares Material auf der äußeren Ringfläche des ersten Ringes (8a) abrollt.

4. Rotationskolbenpumpe nach einem der vorherigen Ansprüche, **dadurch gekennzeichnet, dass** die magnetisch wechselwirkenden Elemente (4), insbesondere ein Ring, der in Umfangsrichtung mehrfach abwechselnd magnetisiert ist, in einem im Kolbenläufer (1) zentrisch rotierbar gelagerten Exzenter (12) angeordnet sind.

5. Rotationskolbenpumpe nach Anspruch 4, **dadurch gekennzeichnet, dass** der Exzenter (12) im Kolbenläufer (1) mittig bezogen auf die axiale Länge des Kolbenläufers (1) angeordnet ist und zumindest zu einer Seite des Exzenters (12), bevorzugt beidseits des Exzenters (12), bevorzugt hinter einer axialseitigen Wandung des Kolbenläufers (1), der Kolbenläufer (1) eine innere Ausnehmung (14) aufweist, in welcher ein motorisch zentrisch rotierbarer Rotor (15) angeordnet ist, der mehrere zu den magnetisch wechselwirkenden Elementen (4) des Kolbenläufers (1) axial beanstandete Permanentmagnete (9) aufweist.

6. Rotationskolbenpumpe nach Anspruch 3 und wenigstens einem der Ansprüche 4 bis 5, **dadurch gekennzeichnet, dass** sich die Rotationswelle des wenigstens einen rotierbaren Rotors (15) durch den ersten magnetischen oder zumindest magnetisierbaren zentrisch an einer axialen Kolbenraumwand befestigten Ring (8a) einer magnetischen Lagerung (8a, 8b) des Kolbenläufers (1) erstreckt.

7. Rotationskolbenpumpe nach einem der vorherigen Ansprüche, **dadurch gekennzeichnet, dass** mehrere Permanentmagnete (4, 4a, 4b) zur Bildung der wechselwirkenden Elemente des Kolbenläufers (1) gebildet sind durch einen permanentmagnetischen Ring, der in Umfangsrichtung mehrfach abwechselnd magnetisiert ist.

## Claims

1. Rotary piston pump, in particular for conveying blood, comprising a piston runner (1), which in particular has two or three lobes in cross section and is arranged in a piston space (2) so as to be driven in an eccentrically rotating manner, wherein the piston runner (1) has in its interior multiple magnetically interacting elements (4, 4a, 4b), in particular permanent magnets and/or coils, and, through magnetic interaction of said elements (4, 4a, 4b), is driven by way of at least one moving magnetic field, **characterized in that** the magnetically interacting elements (4) in the piston runner (1) are arranged in the region of at least one axial end in or below an axial end surface, and multiple coils (5, 5a, 5b), which are able to be energized in an alternating manner for the purpose of generating the moving magnetic field, are arranged behind an axial end wall of the piston space (2) so as to be axially spaced apart from the magnetically interacting elements (4, 4a, 4b) of the piston runner (1), wherein the coils (5, 5a, 5b) are arranged in a manner corresponding to the cross-sectional contour of the shell surface (3) or in a manner corresponding to the movement path of the magnetically interacting elements (4, 4a, 4b) of the piston runner (1).

2. Rotary piston pump according to one of the preceding claims, **characterized in that** the piston runner (1) is mounted in a magnetically eccentrically rotatable manner at at least one of its axial ends.

3. Rotary piston pump according to Claim 2, **characterized in that** the mount is formed by a first magnetic or at least magnetizable ring (8a), which is fastened centrally to an axial piston space wall, and a second magnetic or at least magnetizable ring (8b), which is arranged eccentrically in the piston runner (1) at the axial end and, during the rotation of the piston runner (1), rolls with its inner ring surface on the outer ring surface of the first ring (8a) directly or indirectly via an in particular magnetizable material under magnetically attractive interaction.

4. Rotary piston pump according to one of the preceding claims, **characterized in that** the magnetically interacting elements (4), in particular a ring which is magnetized multiple times in an alternating manner in a circumferential direction, are arranged in an eccentric (12) which is mounted in the piston runner (1) in a centrally rotatable manner.

5. Rotary piston pump according to Claim 4, **characterized in that** the eccentric (12) in the piston runner (1) is arranged centrally in relation to the axial length of the piston runner (1), and, at least on one side of the eccentric (12), preferably on both sides of the eccentric (12), preferably behind an axial wall of the piston runner (1), the piston runner (1) has an inner cutout (14) in which a rotor (15) which is centrally rotatable by motor is arranged, said rotor having multiple permanent magnets (9) which are axially spaced apart from the magnetically interacting elements (4) of the piston runner (1).

6. Rotary piston pump according to Claim 3 and at least one of Claims 4 and 5, **characterized in that** the rotary shaft of the at least one rotatable rotor (15) extends through the first magnetic or at least magnetizable ring (8a), fastened centrally to an axial piston space wall, of a magnetic mount (8a, 8b) of the piston runner (1).

7. Rotary piston pump according to one of the preceding claims, **characterized in that** multiple permanent magnets (4, 4a, 4b) for forming the interacting elements of the piston runner (1) are formed by a permanently magnetic ring, which is magnetized multiple times in an alternating manner in a circumferential direction.

## Revendications

1. Pompe à piston rotatif, notamment destinée au transport de sang, comprenant un rotor de piston (1), notamment à deux ou trois lobes en section transversale, qui est disposé dans un espace de piston (2) en étant entraîné en rotation excentrique, le rotor de piston (1) possédant à l'intérieur de celui-ci plusieurs éléments (4, 4a, 4b) d'interaction magnétique, notamment des aimants permanents et/ou des bobines, et étant entraîné par l'interaction magnétique de ces éléments (4, 4a, 4b) avec au moins un champ magnétique mobile, **caractérisée en ce que** les éléments (4) d'interaction magnétique dans le rotor de piston (1) sont disposés dans la zone d'au moins une extrémité axiale dans ou sous une face frontale axiale et plusieurs bobines (5, 5a, 5b), pouvant être alimentées électriquement en alternance pour générer le champ magnétique mobile, sont disposées espacées axialement par rapport aux éléments (4, 4a, 4b) d'interaction magnétique du rotor de piston (1) derrière une paroi frontale axiale de l'espace de piston (2), les bobines (5, 5a, 5b) étant disposées en correspondance avec le contour de section transversale de l'enveloppe (3) ou en correspondance avec la trajectoire de mouvement des éléments (4, 4a, 4b) d'interaction magnétique du rotor de piston (1).

2. Pompe à piston rotatif selon l'une des revendications précédentes, **caractérisée en ce que** le rotor de piston (1) est supporté magnétiquement à au moins une de ses extrémités axiales de manière à pouvoir effectuer une rotation excentrique.

3. Pompe à piston rotatif selon la revendication 2, **caractérisée en ce que** le palier est réalisé par une première bague (8a) magnétique ou au moins magnétisable fixée de manière centrée à une paroi axiale de l'espace de piston et une deuxième bague (8b) magnétique ou au moins magnétisable disposée de manière excentrique à l'extrémité axiale dans le rotor de piston (1), laquelle, lors de la rotation du rotor de piston (1), roule avec sa surface de bague intérieure, avec une interaction magnétique d'attraction, directement ou indirectement par le biais d'un matériau notamment magnétisable sur la surface de bague extérieure de la première bague (8a).

4. Pompe à piston rotatif selon l'une des revendications précédentes, **caractérisée en ce que** les éléments (4) d'interaction magnétique, notamment une bague qui est magnétisée plusieurs fois en alternance dans la direction périphérique, sont disposés dans un excentrique (12) monté à rotation centrée dans le rotor de piston (1).

5. Pompe à piston rotatif selon la revendication 4, **caractérisée en ce que** l'excentrique (12) est disposé dans le rotor de piston (1) au centre en référence à la longueur axiale du rotor de piston (1) et le rotor de piston (1) possède au moins vers un côté de l'excentrique (12), de préférence des deux côtés de l'excentrique (12), de préférence derrière une paroi côté axial du rotor de piston (1), une cavité interne (14) dans laquelle est disposé un rotor (15) pouvant être mis en rotation centrée de manière motorisée, lequel possède plusieurs aimants permanents (9) espacés axialement par rapport aux éléments (4) d'interaction magnétique du rotor de piston (1).

6. Pompe à piston rotatif selon la revendication 3 et au moins l'une des revendications 4 et 5, **caractérisée en ce que** l'arbre de rotation de l'au moins un rotor (15) rotatif s'étend à travers la première bague (8a), magnétique ou au moins magnétisable et fixée de manière centrée à une paroi d'espace de piston axiale, d'un palier magnétique (8a, 8b) du rotor de piston (1).

7. Pompe à piston rotatif selon l'une des revendications précédentes, **caractérisée en ce que** plusieurs aimants permanents (4, 4a, 4b) sont formés par une bague à magnétisme permanent en vue de former les éléments d'interaction magnétique du rotor de piston (1), laquelle est magnétisée plusieurs fois en alternance dans la direction périphérique.
